# EUROPEAN PATENT APPLICATION

(11) **EP 4 195 083 A1**
(43) Date of publication of application: **14.06.2023**
(21) Application number: 21858089.2
(22) Date of filing: 16.07.2021
(51) Int. Cl.: G06F 21/62, G06Q 50/26, G06F 16/903, G06F 13/00

(54) **USER TERMINAL FOR CONTROLLING AMOUNT OF PERSONAL INFORMATION TO BE EXTERNALLY OUTPUTTED**

(30) Priority: 21.08.2020 JP 2020140195; 12.10.2020 JP 2020171948
(71) Applicant: TESNOLOGY Inc., Minato-ku Tokyo 107-0052 (JP)
(72) Inventor: KONDO Katsuhiko, Tokyo 107-0052 (JP); P. JOHNSON III Raymond, Tokyo 107-0052 (JP)
(74) Representative: Schröer, Gernot H.
(86) International application number: PCT/JP2021/026763
(87) International publication number: WO 2022/038940

(57) **Abstract**

A user terminal 100a stores therein user's personal information I1. The user terminal 100a limits the range of information that can be provided to a user terminal 100b in the personal information I1 to personal information I1b and limits the range of information that can be provided to a user terminal 100c in the personal information I1 to personal information I1c. Similarly, each of the user terminals 100b and 100c also limits the range of information that can be provided in accordance with a partner. Each user terminal 100 may limit the range of information that can be transmitted or received by an output filter or an input filter.

## Description

### Technical Field

The present invention relates to a technique for managing personal information.

### Background Art

In order to deploy unique services, there is an increasing need to frequently process a diverse and large amount of data called "big data". In the future, the need for big data is expected to further increase with the progress of artificial intelligence (AI) or digital transformation (DX) (refer to Patent Literature 1).

### Related Art List

Patent Literature 1: JP 6323334 B

### Summary

### Technical Problem

Meanwhile, it is pointed out that a business strategy in which a large amount of information is collected and enclosed by a company is stuck in a deadlock and has a harmful effect. First, there is a strong repulsive feeling to information control by a huge digital platform operator. The digital platform operator collects an enormous amount of personal information and uses it exclusively to ensure superiority. Individuals do not have the ability to individually negotiate with the digital platform operator and are exposed to a risk of unilateral change to various terms of use of personal information. With an increased awareness of the problem described above, governments are increasingly restricting the use of personal information. General Data Protection Regulation (GDPR) of the EU is a representative example thereof.

There are also energy issues. It is expected that an enormous power demand for data management will be generated in the IoT era in which things and the Internet are connected to each other. While the annual global power consumption in 2016 is about 23,000 TWh, it is predicted to be more than 10 times this amount in 2040. This is because the power consumption of communication networks and data centers is boosted as a result of drastic increase in the number of IoT devices and operations of an enormous number of IoT devices based on the cloud services.

The present invention has been accomplished based on the awareness of the problems described above, and a main object thereof is to propose a technique for allowing an individual to proactively manage his own personal information, in particular, efficiently control the amount of personal information to be provided to outside.

### Solution to problem

A user terminal according to one aspect of the present invention comprises: a data storage unit for storing therein first personal information related to a first user and including a plurality of elements of attribute information; an output setting unit for selecting, as an object of transmission, one or more elements of attribute information from the elements of attribute information included in the first personal information to generate second personal information including the one or more elements of attribute information; and a data transmission unit for transmitting the second personal information selected as the object of transmission from the first personal information.

A user terminal according to another aspect of the present invention comprises: a filter storage unit for storing therein an input filter specifying, as an object to be acquired, one or more elements of attribute information from a plurality of elements of attribute information; a data storage unit for storing therein second external information including one or more elements of attribute information; a data reception unit for receiving first external information from an external device; and an input setting unit for selecting, as an object to be acquired, one or more elements of attribute information from a plurality of elements of attribute information included in the first external information by the input filter to generate the second external information including the selected one or more elements of attribute information and for selecting the second external information as an object to be saved in the data storage unit.

### Advantageous Effects of Invention

According to the present invention, it becomes easy to manage personal information of each person on personal responsibility and to control the amount of personal information to be provided to outside.

### Brief Description of Drawings

FIG. 1 is a concept diagram of information management according to an embodiment.
FIG. 2 is a functional block diagram of a user terminal.
FIG. 3 is a concept diagram of personal information stored in a data storage unit.
FIG. 4 is a data structure diagram of filter setting information.
FIG. 5 is a concept diagram for explaining a method of filtering personal information.
FIG. 6 is a concept diagram when a service is provided in accordance with personal information.
FIG. 7 is a schematic diagram for explaining a first filter setting method.
FIG. 8 is a schematic diagram for explaining a second filter setting method.
FIG. 9 is a schematic diagram for explaining a third filter setting method.
FIG. 10 is a concept diagram of a clothing delivery system according to the embodiment.

### Description of Embodiments

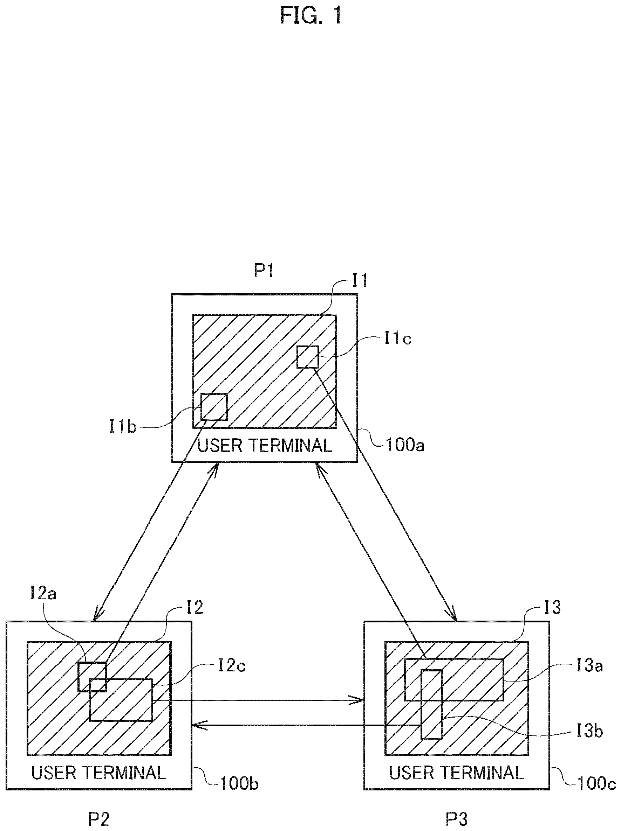

FIG. 1 is a concept diagram of information management in the present embodiment.

In FIG. 1, a user P1, a user P2, and a user P3 own user terminals 100a, 100b, and 100c, respectively. The user terminals 100a to 100c (hereinafter, collectively "user terminals 100") are mobile terminals such as smartphones.

The user terminal 100a stores therein personal information I1 of the user P1. The user terminal 100b stores therein personal information I2 of the user P2. The user terminal 100c stores therein personal information I3 of the user P3. Each of the personal information I1 to I3 is a set of data related to a corresponding user, for example, user's name, age, hobbies and the like. As a general rule, each user manages his personal information in his own user terminal 100, not in the cloud (in a data center), and provides a part of the personal information to outside as necessary. The range of the personal information to be provided to outside is set by each user.

The user P1 limits the range (the type and the amount of personal information) of information that can be output to the other user terminals 100 in the personal information I1. In FIG. 1, the user P1 sets personal information I1b that is a part of the personal information I1 as information that can be transmitted to the user terminal 100b (the user P2) and sets personal information I1c that is another part of the personal information I1 as information that can be transmitted to the user terminal 100c (the user P3). The personal information I1b and the personal information I1c may be the same as each other or different from each other. Further, the amount of information of the personal information I1b and that of the personal information I1c may be the same as each other or different from each other.

In the following descriptions, the user terminal 100 of the user P1 is referred to as "user terminal 100(P1)".

Similarly, in the personal information I2 related to the user P2, personal information I2a is transmitted to the user terminal 100a(P1), and personal information I2c is transmitted to the user terminal 100c(P3) . In the personal information I3 related to the user P3, personal information I3a is transmitted to the user terminal 100a(P1), and personal information I3b is transmitted to the user terminal 100b(P2).

As described above, the user P1 provides only a part of the personal information I1 to the users P2 and P3. The user P1 protects his privacy by limiting the information that can be provided to the other two users from the personal information I1. The user P3 may provide a relatively large amount of information to the users P1 and P2. The user P2 may provide a relatively small amount of information to the user P1 and provide a relatively large amount of information to the user P3. In this manner, each user can set the amount of personal information to be provided from among his personal information in accordance with a providing destination (recipient) in advance.

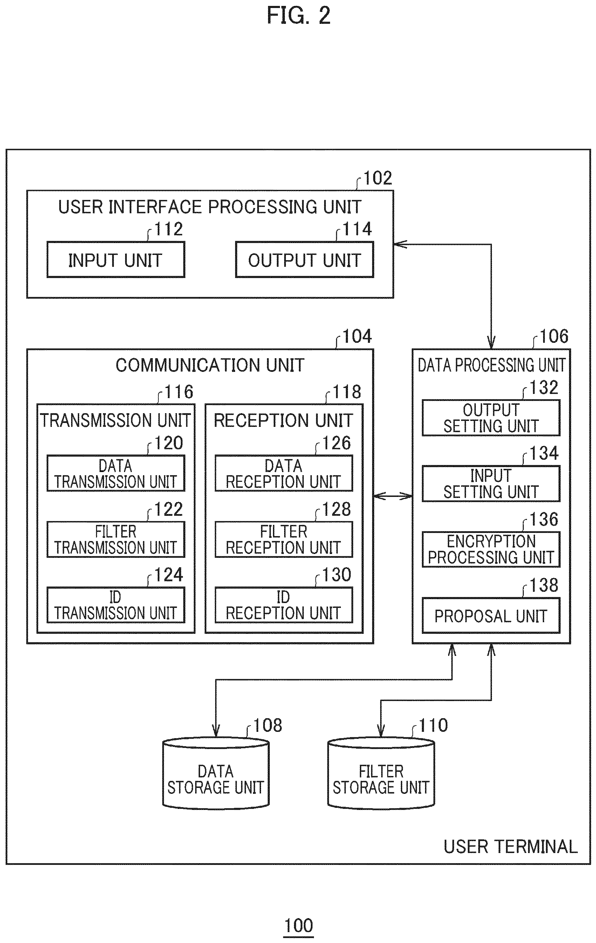

FIG. 2 is a functional block diagram of the user terminal 100.

The constituent elements of the user terminal 100 are implemented by hardware including computing units such as CPUs (Central Processing Units) and various co-processors, storage devices such as memories and storages, and wired or wireless communication lines that couple these units and devices, and software that is stored in the storage devices and supplies processing instructions to the computing units. A computer program may be constituted by one or more device drivers, an operating system, various application programs on upper layers thereof, and a library that provides common functions to these programs.

Blocks described below do not refer to configurations in units of hardware but to blocks in units of functions. In the present embodiment, it is assumed that each function illustrated in FIG. 2 is realized by installing dedicated application software for managing data into a general smartphone.

The user terminal 100 includes a user interface processing unit 102, a communication unit 104, a data processing unit 106, a data storage unit 108, and a filter storage unit 110.

The user interface processing unit 102 performs processes related to user interfaces, such as a process of accepting operations made by a user, an image display process, and an audio output process. The communication unit 104 performs a process of communicating with the other user terminals 100 and the like via a wireless communication network. The data storage unit 108 stores therein various types of personal information (data). The filter storage unit 110 stores filters therein. In personal information, the range of data to be output or input is controlled by various filters (described later). The data processing unit 106 performs various processes based on data acquired by the user interface processing unit 102 and the communication unit 104, data stored in the data storage unit 108, and the filters stored in the filter storage unit 110. The data processing unit 106 also functions as an interface of each of the user interface processing unit 102, the communication unit 104, the data storage unit 108, and the filter storage unit 110.

The user interface processing unit 102 includes an input unit 112 and an output unit 114. The input unit 112 accepts various operations made by a user. The output unit 114 outputs various types of information by image, sound, or the like.

The communication unit 104 includes a transmission unit 116 that transmits various types of information to an external device such as the other user terminal 100 and a reception unit 118 that receives various types of information from the external device.

The transmission unit 116 includes a data transmission unit 120, a filter transmission unit 122, and an ID transmission unit 124. The data transmission unit 120 transmits personal information stored in the data storage unit 108 to the external device. The filter transmission unit 122 transmits a filter stored in the filter storage unit 110 to the external device. The ID transmission unit 124 transmits an ID for identifying the user terminal 100 or a user who owns that user terminal 100 (hereinafter, "user ID") to the external device.

The reception unit 118 includes a data reception unit 126, a filter reception unit 128, and an ID reception unit 130. The data reception unit 126 receives various data (contents) including personal information from the external device. The filter reception unit 128 receives a filter from the external device. The ID reception unit 130 receives a user ID (a destination ID) from the external device, in particular, the user terminal 100 with which data communication is performed.

The data processing unit 106 includes an output setting unit 132, an input setting unit 134, an encryption processing unit 136, and a proposal unit 138.

The output setting unit 132 selects personal information that can be transmitted to the external device from the personal information stored in the data storage unit 108 in accordance with the setting of an output filter (described later). In the following descriptions, personal information of a user himself as a whole stored in the data storage unit 108 is referred to as "first personal information", and personal information in the first personal information which is transmitted to the external device from the data transmission unit 120 is referred to as "second personal information".

The input setting unit 134 selects acceptable personal information from personal information transmitted from the other user terminal 100 in accordance with the setting of an input filter (described later). For example, the user P2 can accept a required (dedicated) range of personal information in personal information transmitted from the user P1 to the user P2, by the input filter. The personal information received from the other user terminal 100 is stored in the data storage unit 108. In the following descriptions, the personal information received from the other user and stored in the data storage unit 108 is referred to as "another person's information".

In the present embodiment, it is assumed that a filter is set by a third setting method described later.

The encryption processing unit 136 encrypts the first personal information and another person's information and stores them in the data storage unit 108. The proposal unit 138 makes various proposals based on the first personal information (all personal information related to the user himself) and another person's information (partial personal information related to another person). Unless otherwise specified, the first personal information, the second personal information, and another person's information are simply referred to as "personal information".

In generation of the second personal information from the first personal information, the encryption processing unit 136 decrypts the first personal information. The output setting unit 132 generates the second personal information from the decrypted first personal information by an output filter. After generation of the second personal information, the encryption processing unit 136 encrypts the first personal information. The data transmission unit 120 transmits the second personal information generated by the output filter to an external device.

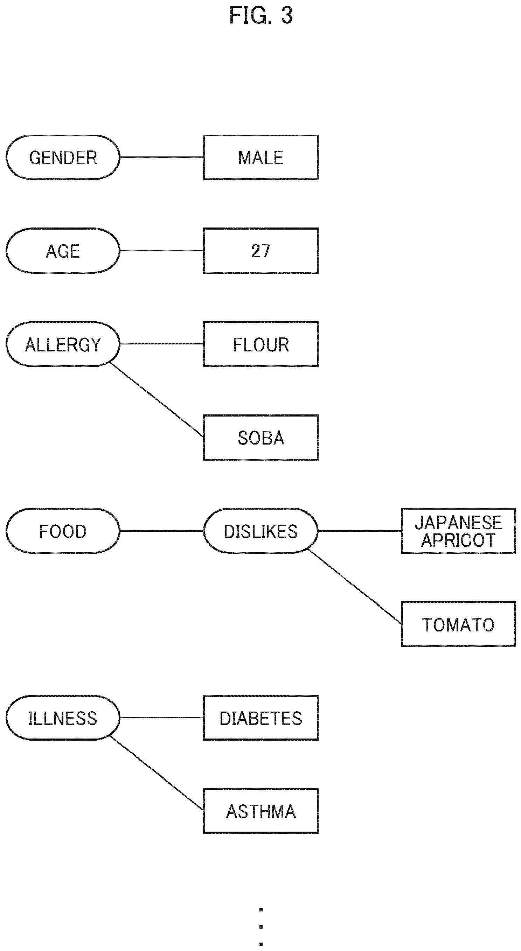

FIG. 3 is a concept diagram of personal information stored in the data storage unit 108.

Personal information is a group of sets of an attribute item (label) and attribute information (content). In FIG. 3, the attribute information of the attribute item "gender" of this user PX is "male", and the attribute information "wheat" and "soba (Japanese buckwheat noodle)" are associated with the attribute item "allergy". Therefore, it can be understood that the user PX is a male and has an allergy to wheat and soba. In FIG. 3, a square with rounded corners indicates an attribute item (label), and a rectangle indicates attribute information (content).

Further, as for the attribute item "food", another attribute item "dislikes" is associated. "Japanese apricot" and "tomato" are associated with the attribute items "food" and "dislikes", as attribute information. It can thus be understood that the user PX does not like Japanese apricots and tomatoes. A plurality of elements of attribute information may be associated with one attribute item, as with the attribute item "allergy". Further, a plurality of elements of attribute information ("Japanese apricot" and "tomato") may be associated with a plurality of attribute items, as with the attribute items "food" and "dislikes".

A user arbitrarily sets attribute information for an attribute item from the input unit 112. The encryption processing unit 136 encrypts new attribute information and saves it in the data storage unit 108 as a part of the first personal information. The encryption processing unit 136 may collectively encrypt the entire first personal information or may encrypt each element of attribute information. Some of many attribute items may be prepared in advance as prescribed attribute items or may be arbitrarily set by the user. Further, the user may also set connection between attribute items arbitrarily.

Input of various types of information from the input unit 112 is not limited to touch input, and may be voice input. In addition, the input unit 112 may detect the body temperature, the heart rate, and/or the odor of the user and register the detection result as personal information. As described above, the input unit 112 may accept input of personal information at any timing via various input interfaces such as a touch panel, a microphone, and a sensor.

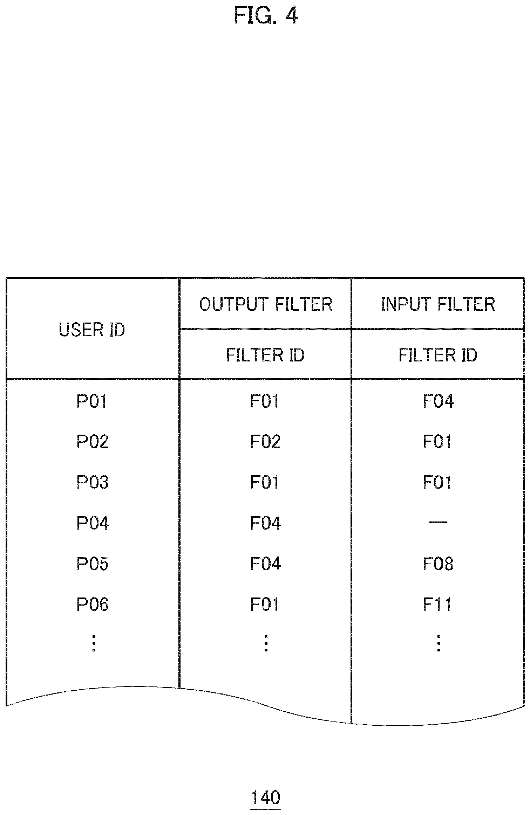

FIG. 4 is a data structure diagram of filter setting information 140.

The filter setting information 140 is stored in the filter storage unit 110 together with various filters. It is assumed that the filter setting information 140 illustrated in FIG. 4 is for a user with a user ID of P11 (hereinafter, "user (P11)"). There are two types of filters: "output filter" and "input filter". The output filter is a filter on an output side (transmission side) for selecting the second personal information that can be provided to outside in the first personal information. The input filter is a filter on an input side (reception side) for selecting acceptable another person's information from the second personal information output from an external device. In the following descriptions, the output filters and the input filters are simply referred to as "filters", unless otherwise specified.

The filters are identified by filter IDs. A user can set any filter. In addition, a user can set any output filter and any input filter in accordance with a communication partner. The filter setting information 140 in FIG. 4 indicates that the user (P11) sets an output filter (F01) for a user (P01). In other words, the user (P11) limits his own personal information by the output filter (F01) and then provides it to the user (P01). In transmission of the personal information from the user (P11) to the user (P01), the output setting unit 132 sets the output filter (F01), and the data transmission unit 120 transmits personal information (the second personal information) of the user (P11) to the user (P01) within a range permitted by the output filter (F01).

The user (P11) sets an input filter (F04) for the user (P01). The user (P11) further limits the personal information permitted to be provided by the user (P01) by the input filter (F04). The input setting unit 134 sets the input filter (F04) when communication with the user (P01) is performed.

The filter setting information 140 in FIG. 4 indicates that the user (P11) sets the output filter (F01) and an input filter (F01) for a user (P03). The output filter and the input filter may be the same filter in this manner. Further, the user (P11) has not set an input filter for a user (P04). Therefore, the user terminal 100 of the user (P11) stores all personal information transmitted from the user (P04) in the data storage unit 108.

For example, when a user terminal 100(P11) and a user terminal 100(P01) communicate with each other, the ID transmission unit 124 of the user terminal 100(P11) transmits the user ID of the user (P11) to the user terminal 100(P01), and the ID reception unit 130 of the user terminal 100(P11) receives the user ID of the user (P01) from the user terminal 100(P01). The output setting unit 132 of the user terminal 100(P11) refers to the filter setting information 140 and sets the output filter (F01) associated with the user (P01) . Similarly, the output setting unit 132 of the user terminal 100(P11) refers to the filter setting information 140 and sets the input filter (F04) associated with the user (P01) . After the above settings are completed, the user terminal 100(P11) and the user terminal 100(P01) start transmission and reception of personal information.

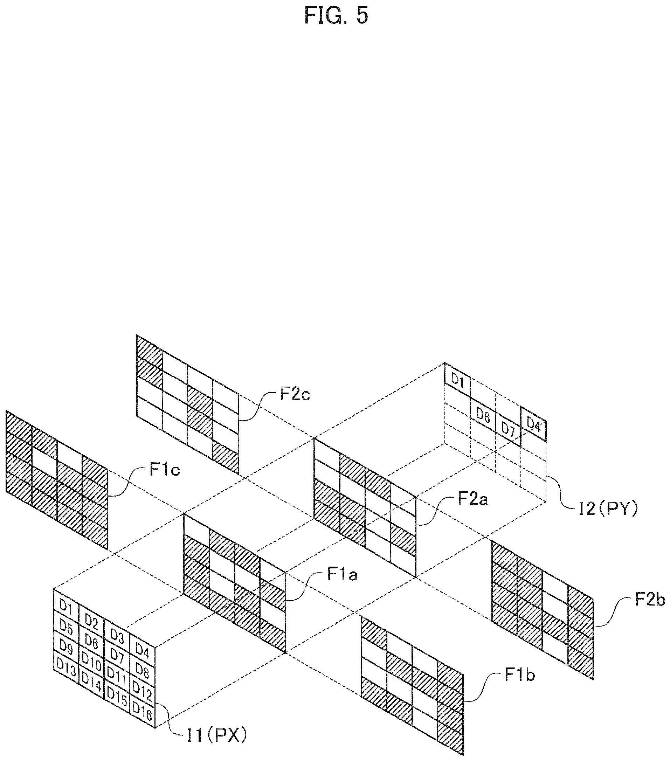

FIG. 5 is a concept diagram for explaining a method of filtering personal information.

Here, a case is described in which personal information (PX) is transmitted from a user terminal 100(PX) of a user PX to a user terminal 100(PY) of a user PY. It is assumed that personal information I1(PX) includes 16 types of data (attribute information) D1 to D16.

The user PX sets a filter F1a as an output filter for the user PY. The user PY sets a filter F2a as an input filter for the user PX. The data D1 reaches the user PY because it is not limited by either the filter F1a or the filter F2a. Meanwhile, data D10 does not reach the user PY because it is limited by the input filter F2a. Data D8 also does not reach the user PY because it is limited by the output filter F1a. Among the data D1 to D16 of the personal information I1, data that can reach the user PY is limited to the data D1, the data D4, the data D6, and the data D7 by the output filter F1a and the input filter F2a.

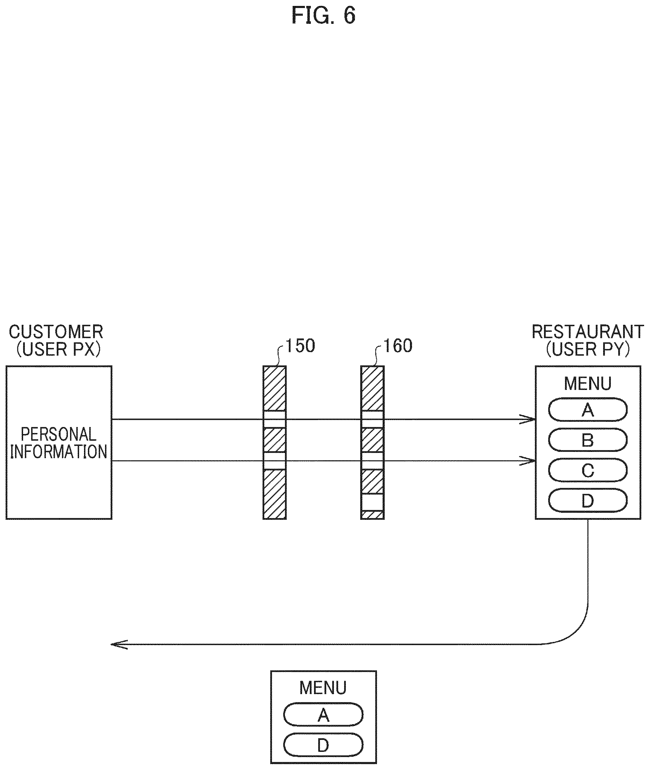

FIG. 6 is a concept diagram when a service is provided in accordance with personal information.

Here, a situation is considered in which the user PX has a meal at a restaurant (user PY). It is assumed that the user PY (restaurant) provides a menu including four dishes: "dish A: ramen", "dish B: fried liver and garlic chives", "dish C: beer", and "dish D: oolong tea", the user PX sets in advance the output filter 150 for the user PY, and the user PY, that is, the restaurant sets in advance the input filter 160 for all users. In the output filter 150, two attribute items, "age" and "do not like" are set as transmittable attribute items. In the input filter 160, three attribute items, "age", "dislikes", and "allergic food" are set as acceptable attribute items.

The data transmission unit 120 transmits only "age: 16" and "dislikes: liver, freshwater fish" in first personal information (PX) to the user terminal 100(PY) as second personal information in accordance with the output filter 150. These elements of information pass through the input filter 160 and are acquired by the user terminal 100(PY) as another person's information.

When the user PX enters this restaurant, the above two types of information are acquired by the user terminal 100(PY) from the user terminal 100(PX). The proposal unit 138 of the user terminal 100(PY) then determines that the dish B (fried liver and garlic chives) and the dish C (beer) are inappropriate in the menu on the basis of another person's information related to the user PX ("age: 16" and "dislikes: liver, freshwater fish"), and generates a menu including only the dishes A and D, and not including the dishes B and C. The data transmission unit 120 of the user terminal 100(PY) transmits the menu thus customized to a monitor on the table of the user PX. According to such a control method, the user PX can select a dish from the menu automatically customized in accordance with the situation or the preference of the user PX even at the restaurant the user PX visits for the first time. Since the user PX provides the minimum personal information to the restaurant (user PY), the user PY can adjust the service in accordance with the user PX.

There are three methods for setting the output filter 150 and the input filter 160. The three setting methods are described assuming that personal data is transmitted and received between the user terminal 100(PX) and the user terminal 100(PY). The present embodiment has been described based on the third setting method described below.

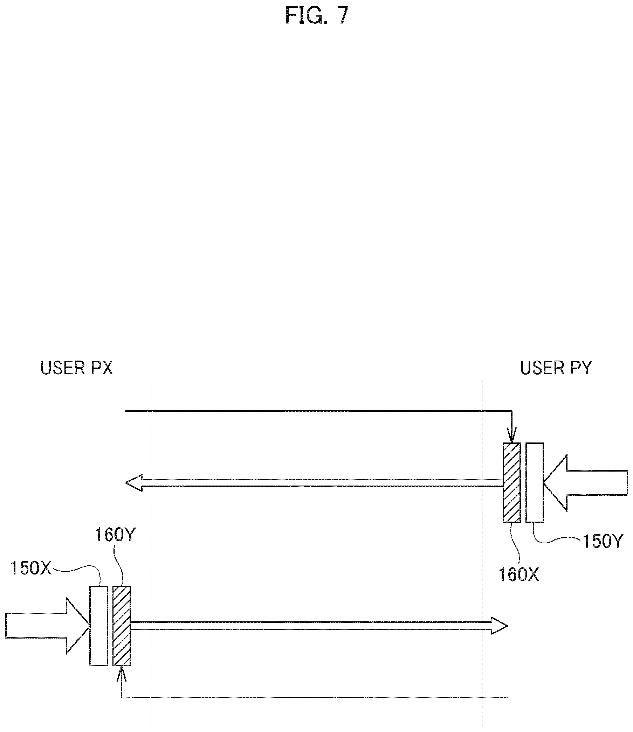

FIG. 7 is a schematic diagram for explaining the first filter setting method.

In the first setting method, after both parties are mutually notified of user IDs, input filters 160X and 160Y are transmitted to the other parties in advance. First, the filter transmission unit 122 of the user terminal 100(PX) transmits the input filter 160X to the user terminal 100(PY). In the user terminal 100(PY), the input setting unit 134 sets the received input filter 160X, and the output setting unit 132 sets the output filter 150Y. Similarly, the filter transmission unit 122 of the user terminal 100(PY) transmits the input filter 160Y to the user terminal 100(PX). In the user terminal 100 (PX), the input setting unit 134 sets the received input filter 160Y, and the output setting unit 132 sets the output filter 150X.

After completion of setting of the input filter 160X, the filter transmission unit 122 of the user terminal 100(PY) notifies the user terminal 100(PX) of completion of filter setting. After completion of setting of the input filter 160Y, the filter transmission unit 122 of the user terminal 100(PX) notifies the user terminal 100(PY) of completion of filter setting. After completion of setting of the input filter 160X and the input filter 160Y are confirmed by both the user terminals 100(PX) and 100(PY), the data transmission unit 120 of the user terminal 100(PY) filters first personal information (PY) by the output filter 150Y and the input filter 160X and transmits the filtering result to the user terminal 100(PX). Similarly, the data transmission unit 120 of the user terminal 100(PX) filters first personal information (PX) by the output filter 150X and the input filter 160Y and transmits the filtering result to the user terminal 100(PY).

In the first setting method, since the other party is caused to set the input filter 160, it is possible to control such that only minimum personal information required flows through a communication path. Consequently, confidentiality of personal information can be enhanced.

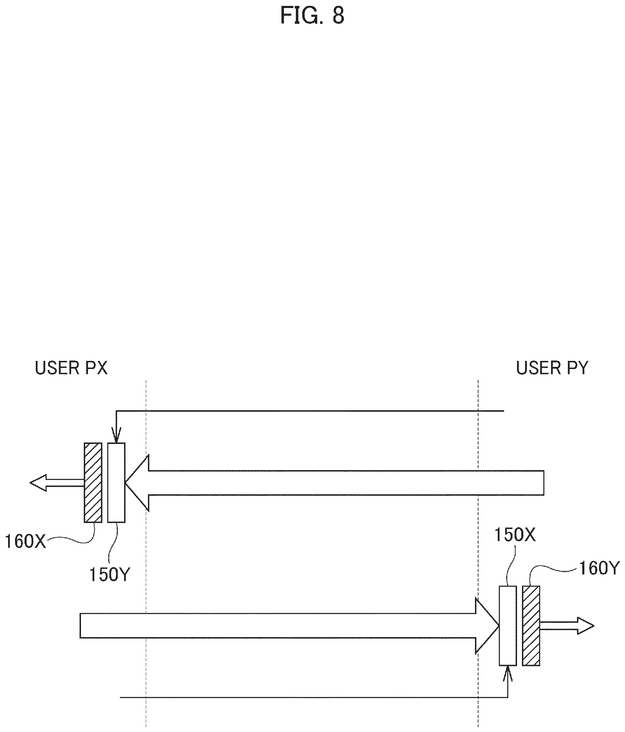

FIG. 8 is a schematic diagram for explaining the second filter setting method.

The filter transmission unit 122 of the user terminal 100(PX) transmits the output filter 150X to the user terminal 100(PY). In the user terminal 100(PY), the output setting unit 132 sets the received output filter 150X, and the input setting unit 134 sets the input filter 160Y. The filter transmission unit 122 of the user terminal 100(PY) transmits the output filter 150Y to the user terminal 100(PX). In the user terminal 100(PX), the output setting unit 132 sets the received output filter 150Y, and the input setting unit 134 sets the input filter 160X. In the second setting method, the reception side sets both the output filter 150 and the input filter 160.

After completion of setting of the output filter 150Y, the filter transmission unit 122 of the user terminal 100(PX) notifies the user terminal 100(PY) of completion of filter setting. Similarly, after completion of setting of the output filter 150X, the filter transmission unit 122 of the user terminal 100(PY) notifies the user terminal 100(PX) of completion of filter setting. After completion of settings of the output filters 150X and 150Y, the data transmission unit 120 of the user terminal 100(PY) transmits the first personal information (PY) to the user terminal 100(PX) as it is. The data transmission unit 126 of the user terminal 100(PX) filters the first personal information (PY) by the input filter 160X and the output filter 150Y to extract another person's information. The same applies to a case in which the user terminal 100(PX) transmits the first personal information (PX) to the user terminal 100(PY).

In the second setting method, since the output filter 150 is set in the other party, the entire first personal information is transmitted to the other party. Therefore, it is desirable that the first personal information is transmitted to the other party after being encrypted by the encryption processing unit 136. In the second setting method, since the output filter 150 is received from a communication partner, the user terminal 100 on the reception side can know what kind of output filter 150 is set by the partner, in other words, what kind of personal information is permitted to be transmitted.

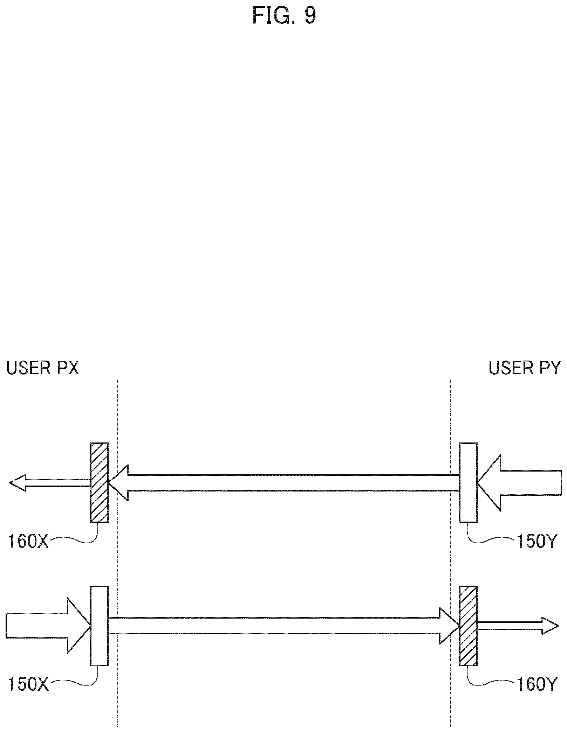

FIG. 9 is a schematic diagram for explaining the third filter setting method.

In the third setting method, transmission and reception of filters is not performed. In the user terminal 100(PY), the output setting unit 132 generates second personal information from first personal information by the output filter 150Y, and the data transmission unit 120 transmits the second personal information to the user terminal 100(PX). The input setting unit 134 of the user terminal 100(PX) generates another person's information from the received second personal information by the input filter 160Y, and stores it for the user (PY) in the data storage unit 108. The same applies to a case of transmitting personal information from the user terminal 100(PX) to the user terminal 100(PY).

In the third setting method, it is unnecessary to transmit a filter to the user terminal 100 as a communication partner in advance. Therefore, the third setting method is advantageous in that it can simplify the communication protocol as compared with the first and second setting methods.

### [Summary]

The method of managing and outputting personal information by the user terminal 100 has been described above by way of the embodiment.

With the user terminal 100, a user on a transmission side can freely set the type and the amount of personal information that can be provided to outside in personal information stored in the data storage unit 108 by the output filter 150. The user can easily register a large amount of personal information related to himself in the user terminal 100 while preventing the personal information from excessively leaking to the outside. Further, the user on the transmission side can control personal information that can be provided, in accordance with a communication partner by changing the output filter 150. Similarly, a user on a reception side can control, by the input filter 160, personal information he wants to receive in personal information provided from the outside. The transmission side and the reception side perform filtering of information the user wants to provide and information the user wants to receive by the output filter 150 and the input filter 160. Even when a large amount of personal information is registered in the data storage unit 108, transmission/reception of personal information is controlled so as to prevent transmission/reception of unnecessary personal information for both parties.

The present invention is not limited to the embodiment described above and a modification thereof and may be embodied by modifying the constituent elements within a range not departing from the scope of the invention. Various inventions may be constituted by appropriately combining the constituent elements disclosed in the embodiment described above and the modification thereof. Further, some constituent elements may be omitted from all the constituent elements disclosed in the embodiment described above and the modification thereof.

### [Modifications]

The present embodiment has been described assuming that dedicated application software for managing data is installed in the user terminal 100 such as a smartphone. In a modification, the function related to personal information management described in the present embodiment (hereinafter, "DI (Digital Intelligence) engine") may be implemented as an LSI (Large Scale Integration) chip (hereinafter, "DI chip"). The DI chip includes a non-volatile memory corresponding to the data storage unit 108 and the filter storage unit 110, software implementing various functions described with regard to the user interface processing unit 102, the communication unit 104, and the data processing unit 106, and an MPU (Micro-Processing Unit) for running this software.

The DI chip may be mounted on the user terminal 100 such as a smartphone. In this case, the DI chip may input and output data via the user interface processing unit 102 of the smartphone. Similarly, the DI chip may transmit and receive data via a communication function of the smartphone. The device on which the DI chip is mounted is not limited to a smartphone, and may be an IC card. The IC card may have a wireless communication function and input and output data via an external reader/writer.

In the present embodiment, the output filter 150 has been described as being changed in accordance with a communication partner. In a modification, a single output filter 150 may be used regardless of the communication partner.

It is not essential to use both the output filter 150 and the input filter 160. The user terminal 100 may use the output filter 150 only or the input filter 160 only. Further, for some communication partners, personal information may be transmitted and received without using the output filter 150 and the input filter 160.

A filter may be changed in accordance with a user's attribute, not depending on a user ID. For example, the user PX may select the output filter 150 and the input filter 160 in accordance with the gender, the age, or the like of the user PY as a communication partner. In one example, the transmission unit 116 of the user terminal 100(PX) transmits an age verification request to the user PY as the communication partner. When the user PY sets the "age" in first personal information as transmittable information, the transmission unit 116 of the user terminal 100(PY) notifies the user terminal 100(PX) of the age of the user PY. The output setting unit 132 of the user terminal 100(PX) selects the output filter 150 in accordance with the age of the user PY. The input filter 160 is also set in a similar manner. According to such a control method, since a filter is changed in accordance with a user's attribute, filter setting can be efficiently performed as compared with a case of setting a filter for each user.

More specifically, a user may set a filter for minors (under ages) and a filter for adults. Alternatively, a user may prepare a filter for students of the same high school and a filter for classmates. Further, a plurality of the output filters 150 may be used at the same time. For example, the possible output range of personal information may be limited by both the two output filters 150, the output filter 150 for students of the same high school and the output filter 150 for classmates. Similarly, a plurality of the input filters 160 may be used at the same time.

The proposal unit 138 may make various proposals in accordance with user's behavioral characteristics. In one example, schedule information of the user PX may be saved in the data storage unit 108 as personal information of the user terminal 100(PX). It is assumed that schedule information "pick up child from cram school by car at 7:00 p.m. on Thursday" is registered for the user PX. It is also assumed that it takes about 20 minutes from the house to the cram school by car. The proposal unit 138 transmits an address 1 that is the house address and an address 2 that is the cram school address to an external traffic information site at 6:00 p.m., that is, one hour before 7:00 p.m., and checks whether traffic congestion has occurred on the route. If traffic congestion has occurred, the proposal unit 138 causes the output unit 114 to output information that "due to traffic congestion, it is advised to leave earlier or take a train to pick up child" to the user PX. According to such a control method, the user PX can be careful in advance not to be late for picking up the child.

The proposal unit 138 of the user terminal 100(PX) transmits limited personal information only including the address 1 of the house and the address 2 of the cram school to the traffic information site in order to check traffic congestion. In other words, it suffices that, with regard to the traffic information site, the output setting unit 132 sets the output filter 150 that allows only two elements of information, the address 1 of the house and the address 2 of the cram school, to be transmitted. In addition to congestion information, it is likely that various types of traffic information such as train operation information and route guidance information are provided from the traffic information site. The input setting unit 134 of the user terminal 100(PX) may set the input filter 160 so as to receive only "congestion information" from the traffic information site. According to such a control method, only by providing minimum personal information required to the traffic information site, it is possible to receive only necessary information such as "traffic congestion".

In the present embodiment, personal information that can be output or received has been described as being limited by masking a part of the personal information with a filter. The personal information may be processed and then provided by providing a converter in addition to the filter. For example, to provide personal information "Okayama University (in Japanese)" to a foreign user, the converter may convert "Okayama University (in Japanese)" to "Okayama University (in English)".

Similarly, the converter may convert an address "Okamoto, Higashinada, Kobe, Hyogo Prefecture" to "Kobe, Hyogo Prefecture", "Hyogo Prefecture", "Kansai region" or "West Japan". Further, the converter may convert the age "43" to "40s", "Around 40", or "adult". As described above, by setting a predetermined converter on an output side or an input side, personal information may be processed and then transmitted and received.

The output setting unit 132 and the input setting unit 134 may perform settings so that only personal information satisfying a predetermined condition can be transmitted and accepted, in addition to using a filter or a converter. For example, the condition may be set in such a manner that the attribute information related to the attribute item "address" cannot be transmitted, or only the attribute information related to the attribute information "Matsuyama" is received.

A filter may be changed depending on a condition other than the user ID or the attribute of a communication partner. The output setting unit 132 and the input setting unit 134 may change the output filter 150 and the input filter 160, respectively, in accordance with a time or a place. The output setting unit 132 may change the output filter 150 depending on whether a user is in Japan or abroad. The output setting unit 132 may change the output filter 150 depending on whether it is morning or afternoon or whether it is a weekday or a weekend.

### [Application of DI engine to clothing industry]

At present, it is said that about 3 billion clothes are produced annually in Japan and about 1.5 billion are discarded. The time from production to disposal (life cycle) of clothing is short, and the environmental load caused by mass production and mass disposal is very high. For example, 10% of global carbon dioxide emissions is said to derive from the clothing industry. Further, in production of clothing, a large amount of water is used for dyeing or the like, and a large amount of water is also discharged. Furthermore, environmental pollution due to release of microplastics is also pointed out.

It is said that one of the reasons for the mass disposal of clothing is an increasingly complex consumption behavior. Customers' preferences for clothing are complex and fluid, and it is very difficult to accurately and properly capture the needs of various customers. This fact is one factor of the mass disposal of clothing.

In a case of operating a store for selling clothing, store maintenance costs are high. While many clothing manufacturers deliver clothing to consumers via E-commerce (electronic commerce) at present, usage fees to be paid to a platform operator providing the E-commerce is also a heavy burden. Not only the production cost of clothing but also the distribution cost of clothing is eventually passed on to the consumers.

Therefore, the inventors of the present invention have considered that by precisely and timely grasping the demands of customers by the DI engines described above, it is possible to bring clothing production as close to made-to-order production as possible and reduce the environmental load and the production cost in the clothing industry.

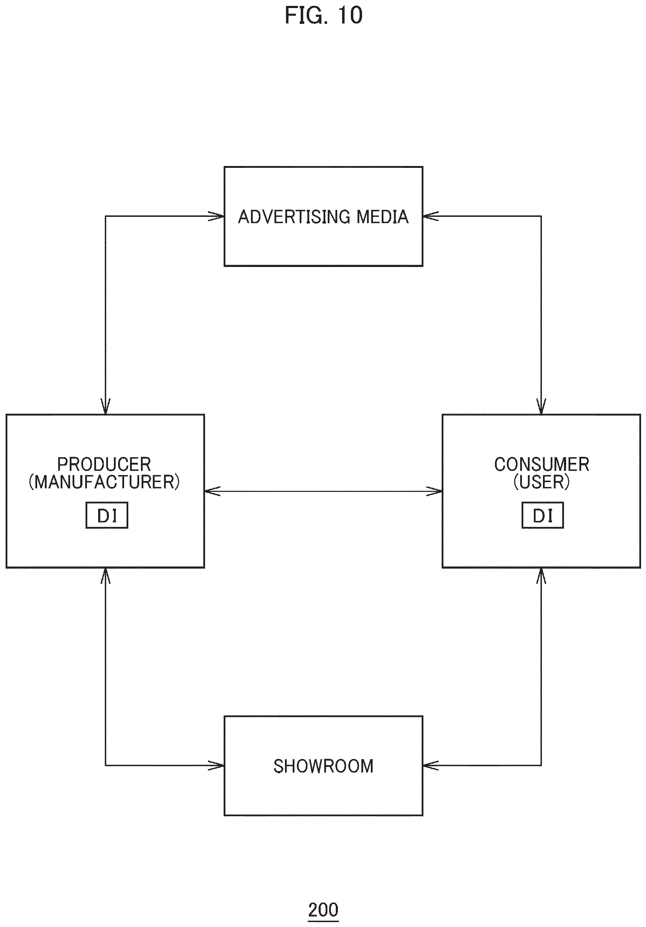

FIG. 10 is a concept diagram of a clothing delivery system 200 in the present embodiment.

First, a DI engine (hereinafter, "user DI") is installed in the user terminal 100 of a user who is a consumer of clothing. A DI engine (hereinafter, "manufacturer DI") is also installed in a server of a manufacturer of clothing. As described above, the user DI stores therein first personal information of the user. The user DI transmits second personal information that is a part of the first personal information to the manufacturer DI. Examples of the second personal information described herein include a body shape such as height and weight, and a favorite color, the brand, and the texture. In addition to the second personal information, the user himself may provide a desired condition related to clothing the user wants. Examples of the desired condition include "summer clothing", "hot-selling line of clothing this year", "within 10,000 yen", "no fur used", and "made in Japan".

The manufacturer DI stores therein a list of clothing that can be provided by the manufacturer. The manufacturer DI selects clothing that matches another person's information and the desired condition provided by the user from the list, and presents the selected clothing to the user DI (user terminal 100). The user selects preferred clothing from the presented clothing and places an order with the manufacturer DI. Alternatively, the manufacturer may design clothing that matches another person's information and the desired condition received from the user DI, present the designed clothing to the user, and start the made-to-order production when the user wants the clothing.

The user pays for the clothing directly to the manufacturer. The manufacturer delivers the clothing to the user. In order to further reduce the financial burden and the environmental load, it is desirable to deliver the clothing by an unmanned carrying device, such as a self-driving car and a drone.

According to such a control method, a clothing manufacturer can propose clothing in accordance with a user's detailed preference. A user is also likely to have the manufacturer propose clothing that matches the user's preference. Since the user and the manufacturer make a direct transaction, it is easy to reduce the distribution cost.

By collecting hobbies and preferences from user DIs of many users, the manufacturer can easily predict what type of clothing is required. Improvement in the prediction accuracy leads to reduction in the amount of discarded clothing. By deeply and quickly grasping customer needs via the user DI, mass production of "unsalable clothing" can be reduced.

The manufacturer may provide information on their clothing on various advertising media. For example, the manufacturer may post an image of clothing on a website operated by a medium such as a magazine and associate product information related to the clothing with the image. Alternatively, the product information may be embedded as a part of image information.

Specifically, the manufacturer first posts image information of clothing A on an advertisement medium (website). At this time, the manufacturer may pay a publishing fee to the operating entity of the website. The user accesses the website and visually recognizes the image of the clothing A. When the user clicks the image of the clothing A, the product information of the clothing A is displayed on the user terminal 100 together with the image of the clothing A. Examples of the product information include the material, the country of origin, the price, the manufacturer name, and the URL of the EC site of the manufacturer.

The user accesses the website (EC site) of the manufacturer by accessing the URL included in the product information of the clothing A and purchases the clothing A directly from the manufacturer. There is another possible flow in which an influencer (a general user) of an SNS introduces the image information and the product information of the clothing A, and the user accesses the image information to know and purchase the clothing A.

Personal preferences for clothing are delicate and complex. It is considered that the manufacturer can not only easily find clothing that matches the user's preference but also easily grasp clothing potentially required by many users by digging up the potential demand from the first personal information of the user by the user DI.

The manufacturer may display clothing in a showroom as is conventional. The user may directly order the clothing displayed in the showroom, or may find out his preference from the clothing displayed and inform the manufacturer DI of his preference via the user DI. In addition, in the showroom, the user may measure the body shape, input the measurement result to the user DI as the first personal information, and transmit it to the manufacturer DI.

### [Reuse of clothing]

Clothing is not only discarded but also distributed as used clothing. It is preferable to reuse clothing as used clothing from the viewpoint of environmental load reduction.

An RFID (Radio Frequency IDentifier) tag may be sewn into clothing. The tag may be a DI chip that aggregates information on the clothing. In the tag (DI chip), information on the clothing, such as the date of manufacture, the material, the owner's attribute, the period of possession, and the country of production, is registered. Examples of the owner's attribute include the name, the gender, the age, and the nationality. For example, it is assumed that a user P1 has purchased clothing B. At this time, the store that sold the clothing B inputs the attribute of the user P1 to the tag of the clothing B. When the user P1 has sold the clothing B to a used clothing store, that store inputs the possession period of the user P1 to the tag of the clothing B. Next, it is assumed that a user P2 has purchased the clothing B at the used clothing store. At this time, the store inputs the attribute of the user P2 to the tag. According to such a control method, by checking the tag (DI chip), a new purchaser can know the distribution process of the clothing B (used clothing). For example, if the clothing B is clothing worn by a famous actor, the value of clothing B may be higher than when the clothing B is new. Used clothing distributed only in Japan may have an "added value" that "the use condition may be good". Used clothing distributed in various countries worldwide may have an "added value" of "being liked in various countries worldwide". Associating information indicating the identity and the history of clothing with the clothing in this manner is considered to contribute to promotion of reuse of clothing.

Instead of embedding a tag in clothing, only a clothing ID may be embedded in the clothing. The clothing ID may be any number as long as it is unique. A server for managing distribution of clothing may be provided, and a distribution process of clothing may be managed by associating information on clothing with clothing ID. Alternatively, a clothing ID and a blockchain may be associated with each other, and owner information may be registered in a new block every time the owner of the clothing changes.

## Claims

1. A user terminal comprising:
a data storage unit for storing therein first personal information that is related to a first user and includes a plurality of elements of attribute information;
an output setting unit for selecting, as an object of transmission, one or more elements of attribute information from the elements of attribute information included in the first personal information to generate second personal information including the one or more elements of attribute information; and
a data transmission unit for transmitting the second personal information generated as the object of transmission from the first personal information.

2. The user terminal according to claim 1, further comprising a filter storage unit for storing therein an output filter specifying, as the object of transmission, the one or more elements of attribute information from the elements of attribute information, wherein
the output setting unit generates the second personal information from the first personal information by the output filter.

3. The user terminal according to claim 2, wherein
the filter storage unit stores therein plural types of the output filters, and
the output setting unit selects one of the output filters in accordance with a destination of the second personal information and generates the second personal information from the first personal information by the selected output filter.

4. The user terminal according to any one of claims 1 to 3, further comprising a filter reception unit for receiving from an external device an input filter specifying, as an object to be acquired, one or more of elements of attribute information from the elements of attribute information, wherein
the output setting unit generates the second personal information from the first personal information by the received input filter, and
the data transmission unit transmits the second personal information to the external device.

5. The user terminal according to claim 1, further comprising:
a filter storage unit for storing therein an output filter specifying, as an object of transmission, one or more elements of attribute information from the elements of attribute information;
a filter reception unit for receiving from an external device an input filter specifying, as an object to be acquired, one or more of elements of attribute information from the elements of attribute information; and
an ID reception unit for receiving a destination ID identifying the external device from the external devices, wherein
the filter storage unit stores therein plural types of the output filters,
the output setting unit generates the second personal information from the first personal information based on both one of the output filters which corresponds to the destination ID and the received input filter, and
the data transmission unit transmits the second personal information to the external device.

6. The user terminal according to any one of claims 1 to 5, further comprising a filter transmission unit for transmitting an input filter specifying, as an object to be acquired, one or more elements of attribute information from the elements of attribute information to an external device.

7. The user terminal according to claim 1, further comprising:
a filter storage unit for storing therein an input filter specifying, as an object to be acquired, one or more elements of attribute information from the elements of attribute information;
a data reception unit for receiving first external information from an external device; and
an input setting unit for selecting, as an object to be acquired, one or more elements of attribute information from a plurality of elements of attribute information included in the first external information by the input filter to generate second external information including the one or more elements of attribute information and for selecting the second external information as an object to be saved in the data storage unit.

8. A user terminal comprising:
a filter storage unit for storing therein an input filter specifying, as an object to be acquired, one or more elements of attribute information from a plurality of elements of attribute information;
a data storage unit for storing therein second external information including one or more elements of attribute information;
a data reception unit for receiving first external information from an external device; and
an input setting unit for selecting, as an object to be acquired, one or more elements of attribute information from a plurality of elements of attribute information included in the first external information by the input filter to generate second external information including the selected one or more elements of attribute information and for selecting the second external information as an object to be saved in the data storage unit.

9. A computer program causing a computer to exert:
a function of storing therein first personal information that is related to a first user and includes a plurality of elements of attribute information;
a function of selecting a part of the elements of attribute information included in the first personal information to generate second personal information including one or more elements of attribute information; and
a function of setting the second personal information as an object of transmission as a part of the first personal information.
